(19) Europäisches Patentamt / European Patent Office / Office européen des brevets

(11) EP 2 601 954 A1

(12) **EUROPEAN PATENT APPLICATION**
published in accordance with Art. 153(4) EPC

(43) Date of publication:
**12.06.2013 Bulletin 2013/24**

(21) Application number: **10855641.6**

(22) Date of filing: **06.08.2010**

(51) Int Cl.:
***A61K 31/555*** *(2006.01)* ***A61K 9/127*** *(2006.01)*
***A61K 9/51*** *(2006.01)* ***A61P 3/10*** *(2006.01)*
***A61P 9/10*** *(2006.01)* ***A61P 25/00*** *(2006.01)*
***A61P 29/00*** *(2006.01)* ***A61P 35/00*** *(2006.01)*
***A61P 39/06*** *(2006.01)*

(86) International application number:
**PCT/JP2010/063396**

(87) International publication number:
**WO 2012/017551 (09.02.2012 Gazette 2012/06)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(71) Applicant: **Yuasa, Makoto**
**Soka-shi, Saitama 340-0021 (JP)**

(72) Inventor: **YUKI Risa**
**Tokyo 164-0012 (JP)**

(74) Representative: **Grünecker, Kinkeldey, Stockmair & Schwanhäusser**
**Leopoldstrasse 4**
**80802 München (DE)**

# (54) THERAPEUTIC AGENT FOR DISEASE

(57) A disease treatment drug having high pharmacological effects and no side effects is provided, the disease treatment drug being capable of intensively delivering an iron or manganese porphyrin complex capable of removing active oxygen to abnormal tissue within a living body, and effectively removing the active oxygen within the abnormal tissue.

The disease treatment drug contains an iron or manganese porphyrin complex nanocapsule housing an iron or manganese porphyrin complex within a nanosized capsule. In the disease treatment drug, disease of abnormal tissue having a high concentration of active oxygen can be treated by the iron or manganese porphyrin complex delivered into the abnormal tissue as a result of the nanocapsule, without affecting normal tissue having a low concentration of active oxygen, and side effects can be suppressed.

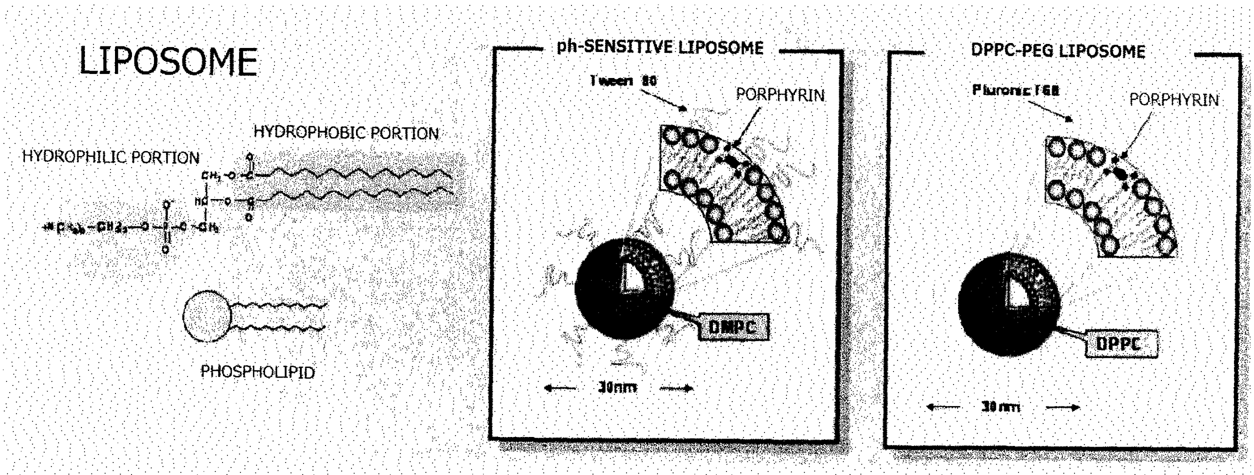


(a) (b) (c)

FIG.1

EP 2 601 954 A1

## Description

TECHNICAL FIELD

The present invention relates to a disease treatment drug used to treat a disease of abnormal tissue within a living body. In particular, the present invention relates to a disease treatment drug that is suitable for treatment of abnormal tissue having a high concentration of active oxygen.

BACKGROUND ART

It is generally thought that the numerous reactive oxygen species generated in the living body contribute to a large number of pathological conditions, such as inflammatory conditions, neurological diseases, arteriosclerosis, cancer, and diabetes. However, in a normal living body, balance is maintained by the presence of radical scavenging enzymes, such as superoxide dismutase (SOD) and catalase, against the reactive oxygen species.

However, it is known that large amounts of superoxide anion radical ($O_2^{-\cdot}$) are present in a cancer cell, which is an example of abnormal tissue in a living body, indicating that enzymatic activities of the radical scavenging enzymes have deteriorated.

On the other hand, diseases such as inflammatory conditions, neurological diseases, arteriosclerosis, and diabetes are also considered to be caused by imbalance of the radical scavenging enzymes, such as superoxide dismutase (SOD) and catalase, leading to increase in reactive oxygen species such as $O_2^{-\cdot}$.

It has been reported that metalloporphyrin complexes show high SOD activity. Therefore, it is anticipated that by administration of metalloporphyrin complexes to the living body, reactive oxygen species such as $O_2^{-\cdot}$ can be effectively removed, and the living body can be protected from biological dysfunctions caused by the active oxygen.

However, numerous issues in terms of safety and effectiveness arise in administering metalloporphyrin complexes by itself to the living body. In actuality, metalloporphyrin complexes have yet to be used as medical drugs.

Therefore, the present applicant has proposed a means for enabling metalloporphyrin complexes to be safely administered to a living body, and SOD activity of the metalloporphyrin complexes to be exhibited (refer to, for example, Patent Literature 1).

Patent Literature 1: Japanese Patent Laid-open Publication No. 2005-041869

DISCLOSURE OF INVENTION

PROBLEM TO BE SOLVED BY THE INVENTION

However, in Patent Literature 1, although the invention is based on experimental proof on a test-tube level, actual pharmacological effects within the living body have not been confirmed.

The present invention has been achieved in light of the above-described issues. An object of the present invention is to provide a disease treatment drug having high pharmacological effects and no side effects, the disease treatment drug being capable of intensively delivering an iron or manganese porphyrin complex capable of removing active oxygen to abnormal tissue within a living body, and effectively removing the active oxygen within the abnormal tissue.

MEANS FOR SOLVING PROBLEM

As a result of keen research, the inventors of the present invention have found further medicinal effects through animal experiments. The inventors have discovered that an iron or manganese porphyrin complex nanocapsule housing an iron or manganese porphyrin complex within a nanosized capsule is not easily delivered to normal tissue within a living body and is predominantly delivered to abnormal tissue because it is a nanocapsule. As a result, active oxygen within the abnormal tissue can be efficiently removed, the disease can be treated, and the abnormal tissue can be returned to normal. Furthermore, they have found that the iron or manganese porphyrin complex is not unnecessarily metabolized by normal tissue or the lymph vessel because of the abnormal tissue that is less developed than the normal tissue. Based on these findings, the inventors have completed the present invention.

In other words, a disease treatment drug according to a first aspect of the present invention is a disease treatment drug containing an iron or manganese porphyrin complex nanocapsule housing an iron or manganese porphyrin complex within a nanosized capsule. In the disease treatment drug, disease of abnormal tissue having a high concentration of active oxygen can be treated by the iron or manganese porphyrin complex delivered into the abnormal tissue

as a result of the nanocapsule, without affecting normal tissue having a low concentration of active oxygen, and side effects can be suppressed.

As a result of a configuration such as this, a "drug delivery system (DDS)" is realized in which a drug is selectively delivered to only an affected area as a result of the drug being in nanocapsule form. The iron or manganese porphyrin complex nanocapsule housing an iron or manganese porphyrin complex is not easily delivered to normal tissue within the living body and can be predominantly delivered to abnormal tissue, thereby efficiently removing active oxygen within the abnormal tissue, treating the disease, and returning the abnormal tissue to normal tissue. In addition, side effects are suppressed as a result.

A disease treatment drug according to a second aspect of the present invention is the disease treatment drug according to the first aspect, in which the porphyrin complex causes a reaction between iron that is in the center and the active oxygen within the abnormal tissue, thereby generating hydrogen peroxide, causes a reaction between the generated hydrogen peroxide and iron, thereby generating hydroxyl radicals, and killing abnormal cells by cytotoxicity of the hydroxyl radicals.

As a result of a configuration such as this, the abnormal cells within the abnormal tissue are killed with certainty by the effect of iron that is bonded in the center of the iron porphyrin complex, and the disease is treated.

In addition, a disease treatment drug according to a third embodiment of the present invention is the disease treatment drug according to the first or second aspect, in which the nanocapsule is composed of a liposome or a polymer capsule.

As a result of a configuration such as this, encapsulation of the iron or manganese porphyrin complex within a nanocapsule can be facilitated.

In addition, a disease treatment drug according to a fourth embodiment of the present invention is the disease treatment drug according to any one of the first to third aspects, in which the nanocapsule has a size of 10nm to 200nm.

As a result of a configuration such as this, the DDS of the iron or manganese porphyrin complex nanocapsule housing an iron or manganese porphyrin complex is realized with certainty, and the disease can be treated with certainty.

## EFFECT OF THE INVENTION

The present invention is configured and works as described above. Therefore, a disease treatment drug having high pharmacological effects and no side effects can be achieved, the disease treatment drug being capable of intensively delivering an iron or manganese porphyrin complex capable of removing active oxygen to abnormal tissue within a living body, and effectively removing the active oxygen within the abnormal tissue.

## BRIEF DESCRIPTION OF DRAWINGS

Fig. 1A, Fig. 1B, and Fig. 1C are explanatory diagrams of an iron or manganese porphyrin complex nanocapsule of the present invention configured as a liposome, in which Fig. 1A shows a phospholipid, Fig. 1B shows a pH-sensitive liposome, and Fig. 1C shows a DPPC-PEG liposome.

Fig. 2A, Fig. 2B, and Fig. 2C are explanatory diagrams of the iron or manganese porphyrin complex nanocapsule of the present invention configured as a polymer capsule, in which Fig. 2A shows a block copolymer, Fig. 2B shows a polymer vesicle composed of a poly(L-lactic acid)-Pluronic F88-poly(L-lactic acid) (PLLA-PluronicF88-PLLA) block copolymer, and Fig. 2C shows a polymer micelle compose of the same.

Fig. 3 is a characteristic line chart of a relationship between the number of days sample drugs are administered to melanoma (skin cancer)-transplanted mice and the rate of increase in tumor volume related to end-stage cancer.

Fig. 4 is a bar graph of the content in Fig. 3.

Fig. 5 is a characteristic line chart of a relationship between the number of days of sample drugs are administered to melanoma (skin cancer)-transplanted mice and weight fluctuations related to end-stage cancer.

Fig. 6 is a characteristic line chart of a relationship between the number of days sample drugs are administered to melanoma (skin cancer)-transplanted mice and the rate of increase in tumor volume related to early-stage cancer.

Fig. 7 is a bar graph of the content in Fig. 6.

## EXPLANATIONS OF LETTERS OR NUMERALS

## BEST MODE(S) FOR CARRYING OUT THE INVENTION

An embodiment of the present invention will hereinafter be described in detail.

A disease treatment drug of the present invention contains an iron or manganese porphyrin complex nanocapsule housing an iron or manganese porphyrin complex within a nanocapsule having a nanosize.

The iron or manganese porphyrin complex nanocapsule will be described.

A liposome and a polymer capsule are used for the nanocapsule.

1) First, the liposome will be described.

In the present specification, an "iron or manganese porphyrin complex-embedded liposome" refers to the iron or manganese porphyrin complex being incorporated in a lipid composing the liposome. A portion of the iron or manganese porphyrin complex is outside of the liposomal membrane or the iron or manganese porphyrin complex is completely enclosed within the liposomal membrane.

The iron or manganese porphyrin complex-embedded liposome of the present invention contains an ionic complex formed by the iron or manganese porphyrin complex and an anionic surfactant (other materials can also be used as the surfactant, as described hereafter), and a lipid having a liposome-forming ability.

The ionic complex formed by the iron or manganese porphyrin complex and the anionic surfactant (hereinafter referred to as simply "ionic complex") that is a constituent of the iron or manganese porphyrin complex-embedded liposome of the present invention is prepared by the surfactant being reacted with the iron or manganese porphyrin complex.

The iron or manganese porphyrin complex which is one of the components forming the ionic complex has a group having a cationic nitrogen atom as a substituent. For example, those expressed by the following formulas (I), (II), and (III) can be given.

[Chemical Formula 1]

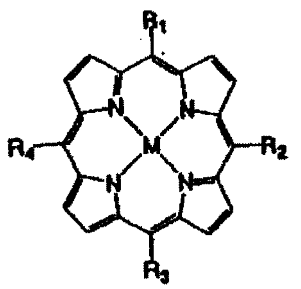

(I)

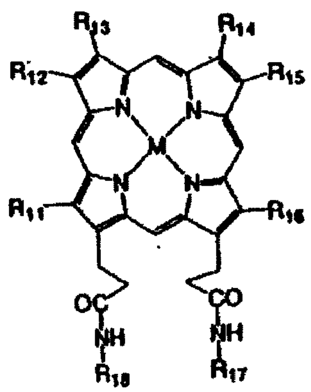

(II)

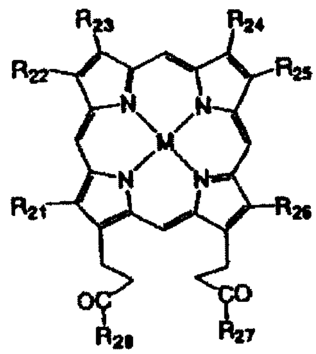

(III)

(In the formula (I), M represents iron or manganese; $R_1$ to $R_4$ each independently represents a group selected from an N-(lower alkyl) pyridyl group, an N-alkyl-ammoniophenyl group, an N-alkyl-imidazolyl group, and a lower di-alkylthiophenyl group; $R_{11}$ to $R_{16}$ each independently represents a lower alkyl group or a lower alkoxy group; $R_{17}$ and $R_{18}$ each independently represents an N-(lower alkyl)pyridyl group, an alkyl-ammoniophenyl group, or an N-alkyl-imidazolyl group, $R_{21}$ to $R_{26}$ each independently represents a low alkyl group or a low alkoxy group, and $R_{27}$ and $R_{28}$ each independently represents an alkyl-ammoniophenyl group).

More specifically, examples can be given in which groups $R_1$ to $R_4$ in the formula (I) are methylpyridyl groups, i.e., 5,10,15,20-tetrakis(2-methylpyridyl)porphyrin (T2MPyP), 5,10,15,20-tetrakis(3-methylpyridyl)porphyrin, and 5,10,15,20-tetrakis(4-methylpyridyl)porphyrin (T4MPyP); groups $R_1$ to $R_4$ are ethylpyridyl groups, ie., 5,10,15,20-tetrakis(2-ethylpyridyl)porphyrin, 5,10,15,20-tetrakis(3-ethylpyridyl)porphyrin, and 5,10,15,20-tetrakis(4-ethylpyridyl)porphyrin; groups $R_1$ to $R_4$ are propylpyridyl groups, ie., 5,10,15,20-tetrakis(2-propylpyridyl)porphyrin, 5,10,15,20-tetrakis(3-propylpyridyl)porphyrin, and 5,10,15,20-tetrakis(4-propylpyridyl)porphyrin; groups $R_1$ to $R_4$ are butylpyridyl groups, ie., 5,10,15,20-tetrakis(2-butylpyridyl)porphyrin, 5,10,15,20-tetrakis(3-butylpyridyl)porphyrin, and 5,10,15,20-tetrakis(4-butylpyridyl)porphyrin; groups $R_1$ to $R_4$ are methylammoniophenyl groups, ie., 5,10,15,20-tetrakis(2-methylammoniophenyl)porphyrin, 5,10,15,20-tetrakis(3-methylammoniophenyl)porphyrin, and 5,10,15,20-tetrakis(4-methylammoniophenyl)porphyrin; groups $R_1$ to $R_4$ are methylimidazolyl groups, ie.,

5,10,15,20-tetrakis(2-methylimidazolyl)porphyrin,
5,10,15,20-tetrakis(3-methylimidazolyl)porphyrin, and
5,10,15,20-tetrakis(4-methylimidazolyl)porphyrin; groups $R_1$ to $R_4$ are dimethylthiophenyl groups, ie.,
5,10,15,20-tetrakis(2-dimethylthiophenyl)porphyrin,
5,10,15,20-tetrakis(3-dimethylthiophenyl)porphyrin, and
5,10,15,20-tetrakis(4-dimethylthiophenyl)porphyrin; groups $R_1$ to $R_4$ are ethylmethylthiophenyl groups, ie.,
5,10,15,20-tetrakis(2-ethylmethylthiophenyl)porphyrin,
5,10,15,20-tetrakis(3-ethylmethylthiophenyl)porphyrin, and
5,10,15,20-tetrakis(4-ethylmethylthiophenyl)porphyrin; groups $R_1$ to $R_4$ are diethylthiophenyl groups, ie.,
5,10,15,20-tetrakis(2-diethylthiophenyl)porphyrin,
5,10,15,20-tetrakis(3-diethylthiophenyl)porphyrin, and
5,10,15,20-tetrakis(4-diethylthiophenyl)porphyrin; and groups $R_1$ to $R_4$ are dipropylthiophenyl groups, ie.,
5,10,15,20-tetrakis(2- dipropylthiophenyl)porphyrin,
5,10,15,20-tetrakis(3- dipropylthiophenyl)porphyrin, and
5,10,15,20-tetrakis(4- dipropylthiophenyl)porphyrin.

In addition, examples can be given in which groups $R_{11}$, $R_{12}$, $R_{14}$, and $R_{16}$ in the formula (II) are methyl, group $R_{13}$ and $R_{15}$ are vinyl, and groups $R_{17}$ and $R_{18}$ are methylpyridyl, i.e., [1,3,5,8-tetramethyl-2,4-divinyl-6,7-di(methylpyridylamidoe thyl)porphyrin (PPIX-DMPyAm); groups $R_{11}$, $R_{12}$, $R_{14}$, and $R_{16}$ are methyl, group $R_{13}$ and $R_{15}$ are vinyl, and groups $R_{17}$ and $R_{18}$ are ammoniophenyl, i.e.,
[1,3,5,8-tetramethyl-2,4-divinyl-6,7-di(ammoniophenylamidoe thyl)porphyrin; groups $R_{11}$, $R_{12}$, $R_{14}$, and $R_{16}$ are methyl, group $R_{13}$ and $R_{15}$ are vinyl, and groups $R_{17}$ and $R_{18}$ are methylimidazolyl, i.e.,
[1,3,5,8-tetramethyl-2,4-divinyl-6,7-di(methylimidazolylami doethyl)porphyrin; groups $R_{11}$, $R_{12}$, $R_{14}$, and $R_{16}$ are methyl, group $R_{13}$ and $R_{15}$ are methoxy, and groups R17 and R18 are methylpyridyl, i.e.,
[1,3,5,8-tetramethyl-2,4-dimethoxy-6,7-di(methylpyridylamid oethyl)porphyrin; groups $R_{11}$ to $R_{16}$ are methyl, and groups $R_{17}$ and $R_{18}$ are methylpyridyl, i.e.,
[1,2,3,4,5,8-hexamethyl-6,7-di(methylpyridylamidoethyl)porp hyrin; and groups $R_{11}$ to $R_{16}$ are ethyl, and groups $R_{17}$ and $R_{18}$ are methylpyridyl, i.e.,
[1,2,3,4,5,8-hexaethyl-6,7-di(methylpyridylamidoethyl)porph yrin.

Furthermore, examples can be given in which groups $R_{21}$, $R_{22}$, $R_{24}$, and $R_{26}$ in the formula (III) are methyl, groups $R_{23}$ and $R_{25}$ are vinyl, and groups $R_{27}$ and $R_{28}$ are methylammonio, i.e., [1,3,5,8-tetramethyl-2,4-divinyl-6,7-di (methylammoniocarbon ylethyl)porphyrin; groups $R_{21}$, $R_{22}$, $R_{24}$, and $R_{26}$ are methyl, groups $R_{23}$ and $R_{25}$ are methoxy, and groups $R_{27}$ and $R_{28}$ are methylammonio, i.e.,
[1,3,5,8-tetramethyl-2,4-dimethoxy-6,7-di(methylammoniocarb onylethyl)porphyrin; groups $R_{21}$ to $R_{26}$ are methyl, and groups $R_{27}$ and $R_{28}$ are methylammonio, i.e.,
[1,2,3,4,5,8-hexamethyl-6,7-di(methylammoniocarbonylethyl)p orphyrin; and groups $R_{21}$ to $R_{26}$ are ethyl, and groups $R_{27}$ and $R_{28}$ are methylammonio, i.e.,
[1,2,3,4,5,8-hexaethyl-6,7-di(methylammoniocarbonylethyl)po rphyrin.

Syntheses of the cationized, cationic porphyrin complexes expressed in the formula (I) in which metal is coordinated, among the examples above, can be conducted in adherence to a process such as that disclosed in K. Kalyanasundaram, Inorg. Chem., 23, 2453 (1984), A.D. Adler et al., J. Inorg. Nucl. Chem., 32, 2443 (1970), T. Yonetani et al., J. Biol. Chem., 245, 2988 (1970), P. Hambright et al., Inorg. Chem., 15, 2314 (1976), M. Antionietti, Langmuir, 16, 3214 (2000), D. Adler et al., J. Org. Chem., 32, 476 (1967), D. Adler et al. Inorg. Synth., 16, 213 (1976), Harriman et al. , J. Chem. Soc., Faraday. Trans. II, 1532 (1979).

Furthermore, syntheses of the cationized, cationic porphyrin complexes expressed in the formulas (II) and (III) in which metal is coordinated can be conducted in adherence to a process such as that disclosed in E. Tsuchida, H. Nishide, H. Yokoyama, R. Young, and C.K. Chang, Chem. Lett., 1984, 991.

The chemical structures of above-described metal[5,10,15,20-tetrakis(2-methylpyridyl)porphyrin] (MT2MPyP) and
metal[5,10,15,20-tetrakis(4-methylpyridyl)porphyrin] (MT4MPyP) are as shown below in chemical formula (2): the chemical structure of MT2MPyP; and chemical formula (3): the chemical structure of MT4MPyP.

[Chemical Formula 2]

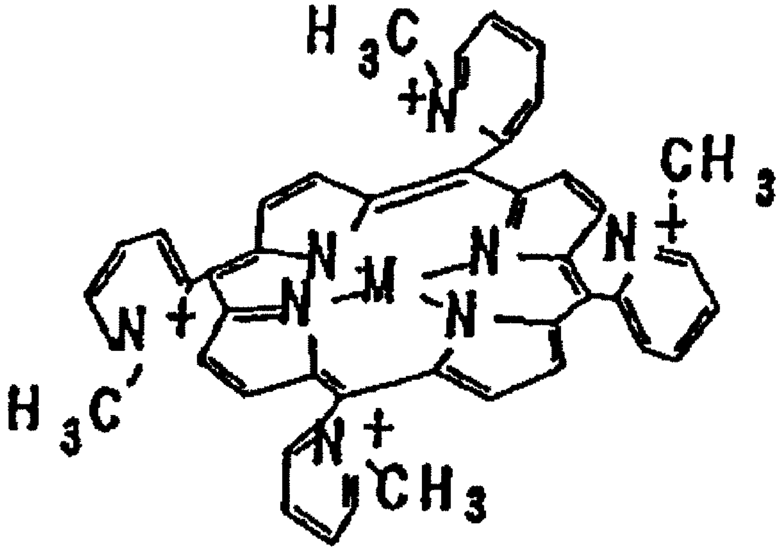

[Chemical Formula 3]

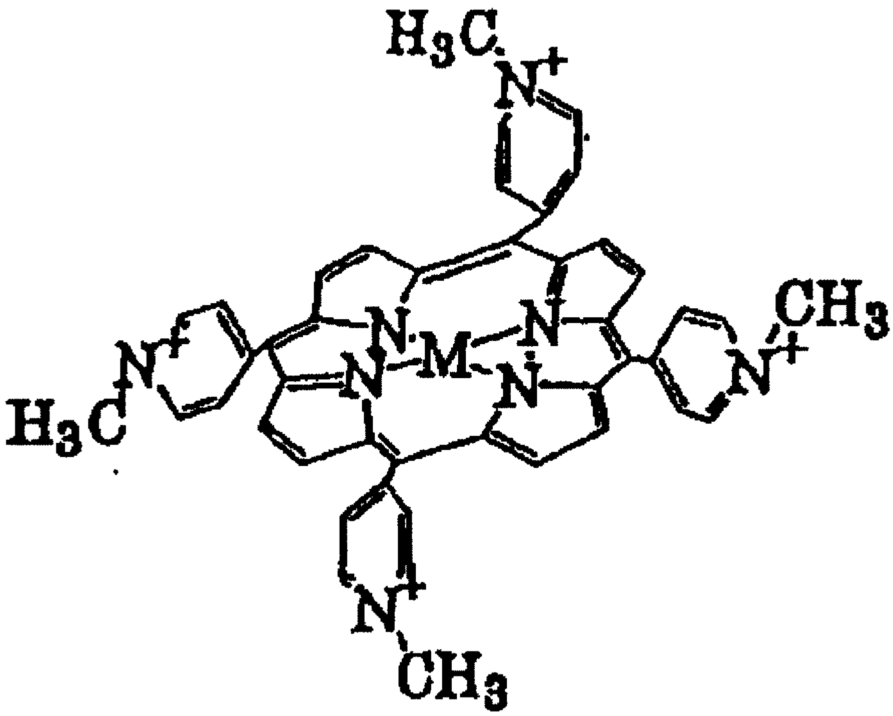

The chemical structure of above-described metal [5,10,15,20-tetrakis(4-dimethylthiophenyl)porphyrin] ($MT4Me_2SuP$) is as shown below in chemical formula (4): the chemical structure of $MT4Me_2SuP$.

[Chemical Formula 4]

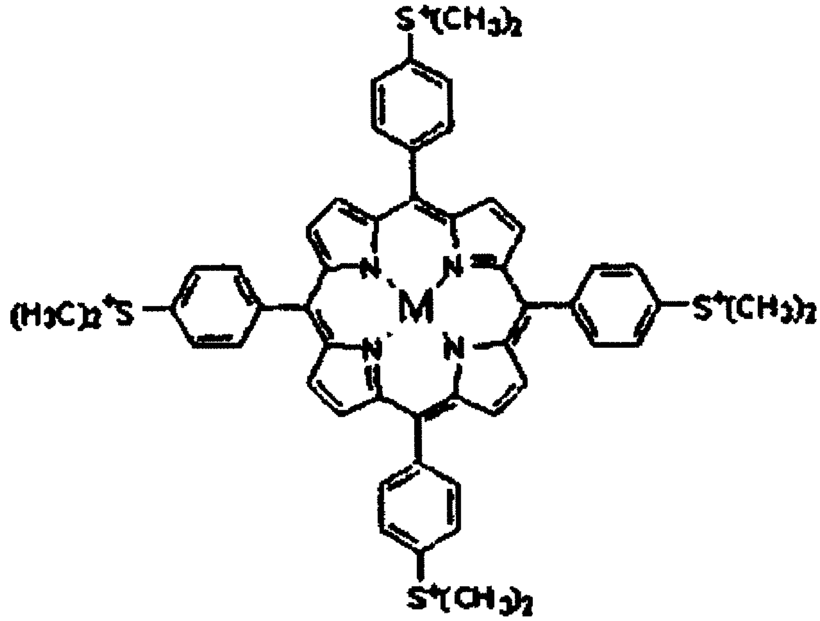

On the other hand, as the anionic surfactant which is another component forming the ionic complex, alkali metal salt of a fatty acid or an alkali metal salt of an alkylsulfuric acid is preferred. As examples of the alkali metal salt, the alkali metal salts of fatty acids, such as lauric acid (LAS), myristic acid (MAS), palmitic acid (PAS), stearic acid (SAS), and oleic acid (OAS), and alkali metal salts of alkylsulfuric acids, such as dodecylsulfuric acid (SDS), tetradecylsulfuric acid (STS), hexadecylsulfuric acid (SHS), and octadecylsulfuric acid (SOS) are given. As the alkali metal salts of fatty acids and the alkali metal salts of alkylsulfuric acids, sodium, potassium, and the like are preferred.

To form the ionic complex, the iron or manganese porphyrin complex is merely required to be mixed with the anionic surfactant in an appropriate solvent. The mixing ratio of the iron or manganese porphyrin complex to the anionic surfactant may be set to about 1:1 or 1:20 in terms of molar ratio.

The ionic complex formed as described above is then mixed with a lipid having liposome-forming ability (hereinafter referred to as a "lipid"), and subsequently formed into the iron or manganese porphyrin complex-embedded liposome by a common method for forming liposomes.

As the lipid, a phospholipid containing, as the sole component, soy lecithin (SBL), egg yolk lecithin (EYL), dilauroyl phosphatidylcholine (DLPC), dimyristoylphosphatidylcholine (DMPC), dipalmitoylphosphatidylcholine (DPPC), distearoylphosphatidylcholine (DSPC), dioleoylphosphatidylcholine (DOPC), monooleoyl-monoalkyl phosphatidylcholine (MOMAPC), or the like, or a matrix containing the phospholipid as a main component in combination with another component (hereinafter also referred to as a "mixed phospholipid") can be given.

As a component which can be mixed with the phospholipid when preparing the mixed phospholipid, fatty acids such as oleic acid (OAS), and surfactants such as dimethylditetradecylammonium bromide (DTDAB), Tween-61 (TW61), and Tween-80 (TW80) can be given.

In particular, liposomes obtained from mixed lipid systems composed of phospholipids such as DMPC and DPPC and cationic surfactants such as DTDAB, dimethyldihexadecylammonium bromide (DHDAB), anionic surfactants such as OAS and SAS, or nonionic surfactants such as TW61 and TW80 are pH-sensitive liposomes. For example, when the pH-sensitive liposome is taken into a cancer cell, deaggregation of the liposome occurs because the pH within the cancer cell is low, thereby enabling a more effective sustained release of an anticancer agent. A system in which an ionic complex is embedded in such pH-sensitive liposome (iron or manganese porphyrin complex-embedded pH-sensitive liposome) can also be synthesized.

As the mixed phospholipid, that in which known cholesterol (Chol) or the like is added to the phospholipid, and that in which polyethylene glycol or a derivative thereof is added to the phospholipid can be given.

To form the iron or manganese porphyrin complex-embedded liposome from the above-described ionic complex and lipid, first, these components are required to be placed in an appropriate solvent and sufficiently mixed.

The amounts of ion complex and lipid to be used when forming the liposome is preferably 10 moles to 500 moles, and particularly 50 moles to 300 moles, of lipid per mole of the ionic complex.

The liposome can be formed by a known method. For example, after both of the above-described components

are dissolved and mixed in a volatile solvent, the volatile solvent alone is stripped and removed. Next, a suitable aqueous solvent, such as purified water or normal saline, is added to the residue, and the resultant is vigorously stirred or undergoes ultrasonication. As a result, the iron or manganese porphyrin complex-embedded liposome is formed.

A solution in which a pharmaceutically effective ingredient is dissolved, a certain type of culture medium, or the like can be used, as required, instead of the aqueous solvent. An iron or manganese porphyrin complex-embedded liposome that includes such solution or medium can be obtained.

The iron or manganese porphyrin complex-embedded liposome obtained as described above may be obtained by structural analysis being performed through use of spectrofluorimetry, dynamic light scattering analysis, or the like (refer to Patent Literature 1).

In addition, the particle size of the liposome is 10nm to 50nm, and is found to be a size capable of reaching cells when taken into the body.

2) Next, the polymer capsule will be described.

The polymer capsule contains a biodegradable polymer as a constituent and is a biodegradable nanocapsule.

The biodegradable nanocapsule has a nanoparticle structure composed of amphiphilic block copolymers. The amphiphilic block copolymers form a self-assembly of nanoparticles having a hydrophobic inner core (core) and a hydrophilic outer shell (shell) in an aqueous solution. Therefore, the amphiphilic block copolymers are applied to a drug delivery system. The structure of the nanoparticles formed by the amphiphilic block copolymers can be varied as a result of the compositions of the hydrophobic and hydrophilic polymer chains being changed. For example, the structure can be spherical, vesicle-shaped, rod-shaped, or tubular. These structures are expected to serve as media for delivering drugs.

Poly(ethylene oxide)-poly(propylene oxide)-poly(ethylene oxide) block copolymer (PEO-PPO-PEO; Pluronic) has excellent biocompatibility, and is a macromolecular surfactant that is being widely researched as a drug carrier. However, as a result of the low-hydrophobic PPO block, the critical micelle concentration (CMC) of Pluronic is generally significantly high. When Pluronic is administered into a living body, there is a disadvantage in that Pluronic becomes unstable due to dilution and is easily disintegrated. It is known that the CMC becomes very low as a result of Pluronic being modified by a hydrophobic polyester, such as polycaprolactam (PCL), polyglycolic acid (PGA), or polyacrylic acid (PAA). Polyester, such as poly(L-lactic acid) (PLLA), PCL, and PGA, has excellent biodegradability and biocompatibility, and is degraded to small molecules to through hydrolysis or enzymolysis. After being administered into a living body, polyester can be discharged outside of the body by renal elimination without accumulation. Therefore, amphiphilic block copolymers containing a hydrophobic polyester portion are gaining attention. Polyester is known to degrade slowly because of its high hydrophobic property. However, reports indicate that the degradability of polyester improves when modified by flexible hydrophilic PEO. Therefore, an amphiphilic block copolymer composed of poly (ethylene oxide) and polyester is expected to serve as a controlled release carrier for drugs and the like, as a result of the block compositions of PEO and polyester being changed.

In the present invention, as a polymer capsule having the functions described above, a PLLA-Pluronic F88-PLLA block copolymer is synthesized in which both ends of Pluronic F88 are modified by a hydrophobic PLLA chain.

The reason for using Pluronic and polylactic acid is as follows. Pluronic is a thermoresponsive amphiphilic polymer, and is one of the very few biocompatible synthetic polymer materials approved by the U.S. Food and Drug Administration. Polylactic acid is a biodegradable and biocompatible ester that, by modifying Pluronic, can improve the hydrophobic property and reduce the CMC of Pluronic. In addition, use in drug-release formulations can be expected as a result of the PLLA blocks.

Synthesis is performed as follows.

With tin(II)2-ethylhexanoate as a catalyst, L-lactide was ring-opening polymerized on both ends of Pluronic F88. As a result, a Pluronic F88/poly(L-lactic acid) block copolymer which is an amphiphilic polymer was synthesized.

Specifically, Pluronic F88 was dried under reduced pressure (12h at 30°C) in advance. L-lactide was heated and dissolved in a mixed solution of dehydrated toluene/dehydrated THF=1/1, recrystallized, and subsequently dried under reduced pressure (12h at 30°C). Tin(II)2-ethylhexanoate was prepared using dehydrated toluene to be 0.1g/ml.

An eggplant-shaped flask with a three-way stopcock was placed in a dryer and moisture was removed. The flask was filled with Ar gas, and predetermined amounts of PEG and L-lactide were placed in the flask (see Table 1). A freezing-thawing process was performed five times, and moisture was completely removed. After the Ar gas had been substituted, predetermined amounts of toluene (2ml in relation to 0. 5g of L-lactide) and an Sn $(Oct)_2$ solution were dripped. The resultant was polymerized for three hours at 135°C and then allowed to cool. The resultant was then dissolved in 30ml of chloroform, reprecipitated in 300ml of diethyl ether, which is ten times the amount of chloroform, and filtered through a membrane filter (pore diameter of 0.1μ). After being dried under reduced pressure, the resultant was dissolved in 30ml of dehydrated chloroform, reprecipitated in 300ml of methanol/diethyl ether mixed solution, and filtered through a membrane filter (pore diameter of 0.1μ). The resultant was dried at a reduced pressure (12h at 30°C) and, a white Pluronic F88/poly(L-lactic acid) block copolymer was obtained.

[Table 1]

TABLE1 PREPARATION VOLUME FOR SYNTHESIS OF PLLA-F88 BLOCK COPOLYMER

| SAMPLE | POLY(L-LACTIC ACID)a) ($W_{LA}$ : wt%) | L-LACTIDE (g) | PLURONIC F88 (g) | $Sn(Oct)_2$b) (μl) | TOLUENE (ml) | METHANOL/ETHER (v/v) |
|---|---|---|---|---|---|---|
| $PLLA_{60wt\%}$-F88 | 60 | 3 | 2 | 450 | 12 | 60/40 |
| $PLLA_{80wt\%}$-F88 | 80 | 4 | 1 | 600 | 16 | 80/20 |

a) INTRODUCTION RATE IN RELATION TO PLURONIC F88 (WLA : wt%). b) 0.1g/ml TOLUENE SOLUTION

A solvent evaporation technique was used in the method for preparing a polymer capsule using the Pluronic F88/poly(L-lactic acid) block copolymer.

The solvent evaporation technique is a method for easily obtaining a polymer solution dispersed in a poor solvent by adding a poor solvent to a polymer solution and gradually evaporating good solvent. As a result of preparation conditions being controlled, the particle size of fine particles can be changed on the nano-scale to the micro-scale. The shape of the fine particles can be controlled not only to be spherical, but also hollow or semi-spherical. In the instance of amphiphilic polymers, self-assembly occurs due to factors such as hydrophobic interactions which are nonspecific interactions, hydrogen bonding and electrostatic interactions which are specific interactions, thereby forming micelles, vesicles, and the like.

Specifically, 10mg of the block copolymer was added to a centrifuging tube and dissolved by 3ml each of tetrahydrofuran or acetone. Then, 10ml of ion-exchange water was added a drop at a time while the solution was being vigorously stirred. The solvent was subsequently evaporated by a rotary evaporator. Pressure reduction operation was continued even when bubbles of tetrahydrofuran or acetone no longer appeared visibly, and the good solvent was completely removed.

Vesicles were formed when tetrahydrofuran was used. Micelles were formed when acetone was used.

The polymer capsule obtained as described above may be obtained by structural analysis being performed through use of spectrofluorimetry, dynamic light scattering analysis, or the like.

The particle size of the polymer capsule is 50nm to 200nm, and is found to be a size capable of reaching cells when taken into the body.

[Examples]

Pharmacological actions and effects of the disease treatment drug of the present invention will hereinafter be described based on animal experiments.

1) Sample Drugs

The iron or manganese porphyrin complex nanocapsules used in the examples were:

1) iron or manganese porphyrin complex/pH-sensitive liposomes;
2) iron or manganese porphyrin complex/DPPC-PEG liposomes;
3) iron or manganese porphyrin complex/$PLLA_{80wt\%}$-F88 vesicles; and
4) iron or manganese porphyrin complex/$PLLA_{60wt\%}$-F88 micelles.

The liposomes of sample drugs 1) and 2) encapsulate the iron or manganese porphyrin complex by a phospholipid shown in Fig. 1A. When DMPC was used as the surfactant, the pH-sensitive liposome shown in Fig. 1B was formed. When DPPC was used as the surfactant, the DPPC-PEG liposome shown in Fig. 1C was formed.

The iron or manganese porphyrin complex/$PLLA_{80wt\%}$-F88 vesicles of sample drug 3) and the iron or manganese porphyrin complex/$PLLA_{60wt\%}$-F88 micelles of sample drug 4) encapsulate the iron or manganese porphyrin complex by the PLLA-F88 block copolymer shown in Fig. 2A. As shown in Fig. 2B, in the vesicle, the iron or manganese porphyrin complex was housed in the membrane portion formed by the PLLA-F88 block copolymer of the membrane vesicle that is a hollow particle. As shown in Fig. 2C, in the micelle, the iron or manganese porphyrin complex was housed in the particle portion formed by the PLLA-F88 block copolymer of the endoplasmatic reticulum that is a particle. ("Hydrophilic drug" and "hydrophobic drug" noted in the drawings indicate a typical housing state of the drugs.)

Cisplatin (CDDP) and mitomycin (MMC) currently used in clinical applications were used as comparison examples.

2) Pathological cells

B16 melanoma cells were used as cells for cancer transplantation experiments to evaluate anticancer pharmacological activities.
The B16 melanoma cells have the following characteristics:

· melanoma cells from C57BL/6 mice;
· cells that have no life span, and infinitely and continuously increase;
· adherent cells having relatively high metastatic ability;
· cells easily coming into contact with air and anchoring onto organs as a result of being skin cancer cells.

Therefore, it has been confirmed that, when the melanoma cells are injected into the tail vein of the mouse for transplantation to the mouse, the cancer anchors onto the lungs.
In addition, the melanoma cells produce melanin and turn black, thereby becoming easy to see and enabling cancer anchoring to be clearly observed. Therefore, the melanoma cells are widely used as model tumor cells.
This melanoma itself has a very low probability of occurring in humans. However, the fatality rate when melanoma occurs is extremely high. Because the malignancy grade of melanoma is the highest of all carcinomas, melanoma is greatly feared. In humans, melanoma is often referred to as "mole cancer" and tends to occur on the sole of the foot.

3) Experimental animals

1) Female, six-week-old C57BL/6CrSlC mice were used to verify the response of end-stage cancer. Although the fur of the mice is black, the foot pads (soles) are flesh-colored. Because the cancer cells are black, growth and regression of the cancer can be accurately observed by sight.
2) Female, six-weeks-old ICR mice were used to verify the response of early-stage cancer. The fur of the mice is white and the foot pads (soles) are flesh-colored. Because the cancer cells are black, growth and regression of the cancer can be accurately observed by sight. Furthermore, fur loss can also be accurately observed by sight.

Example 1 (End-stage cancer)

1) Animals

Five female, six-week-old C57BL/6CrSlC mice were assigned per group.

2) Sample drugs

As the sample drugs (concentration) of the present invention,
iron porphyrin complex/pH-sensitive liposomes (5mM/36mM) and
iron porphyrin complex/DPPC-PEG liposomes (5mM/36mM) were used
As the sample drug (concentration) of the comparative example,
MMC (0.9mM) was used.

3) Cancer cells

B16 melanoma cells were used.

4) Testing method

B16 melanoma dispersed in PBS was injected into the foot pads (sole) of the mice (C57BL/6, female, six-weeks-old), and cancer was transplanted. The amount of cancer cells injected was $1\times10^6$/mouse/0.05ml. The site of injection was within the limited area of the foot pad, in an environment where numerous fine blood vessels, such as capillaries, are present.
Anchoring of the cancer was confirmed on the 10th day after cancer cell transplantation. The mice were separated into five mice each for the required number of groups (four groups: three groups for the sample drugs and one control group). At the same time, the short diameter and the long diameter of the cancer cell volume in each mouse were measured every two days using calipers. The following equation was calculated:

$$[\text{tumor volume}]=1/2\times[\text{long diameter}]\times[\text{short diameter}]^2$$

In addition, administration of the sample drugs (administered amount: 0.1ml/mouse/dose) was started on the 13th day after cancer cell transplantation. Drug administration was conducted a total of four times, via tail vein every four days. The weight of each mouse was measured every two days.

5) Results

The rate of increase in tumor volume was as shown in Fig. 3 and Fig. 4. Weight fluctuation was as shown in Fig. 5.

Based on the above-described results, it is clear that the iron porphyrin complex can reduce side effects and exhibit overwhelming carcinostatic action as a result of being embedded in the liposome.

The iron porphyrin complex embedded in the liposome has higher antitumor activity compared to MMC which is a commercially available anticancer agent. In this instance, although the drug concentration of MMC was merely 0.5mM, two mice died during 20 days of observation. Regarding weight increase and decrease as well, whereas severe weight fluctuations occurred as a result of side effects of MMC, weight was relatively stable for the iron porphyrin complex of the present invention (see Fig. 5). Therefore, it can be said that the iron porphyrin complex has few side effects attributed to the drug, while having very high carcinostatic action, and can be strongly expected to serve as a carcinostatic agent. A reason for this is because, while MMC exhibits pharmacological effects by acting on the DNA itself, the iron porphyrin complex has a specific carcinostatic mechanism. In other words, the iron porphyrin complex targets the active enzymes being specifically abnormally produced only in cancer cells, and kills the cancer through necrosis by producing hydrogen peroxide from active oxygen, and further producing hydroxyl radicals through Fenton reaction. Therefore, cytotoxicity to normal cells that produce less active oxygen than cancerous tissue is low. As a result, the side effects are clearly significantly fewer compared to those of MMC. Furthermore, porphyrin itself is considered to have 30 times the cancer accumulation action of typical anticancer agents. Therefore, porphyrin actively accumulates in the cancerous tissue and is effective even when administered by itself.

Furthermore, significant carcinostatic action was found in mice that had been administered the drug in which the iron porphyrin complex is enclosed in pH-sensitive liposome, compared to the control group. In one mouse, complete remission (complete elimination of cancer cells) was seen after six days.

In addition, significant carcinostatic action was similarly seen in the system enclosed in the DPPC-PEG liposome.

On the other hand, regarding the control group (administered MMC, necrosis occurred in the foot itself, and sudden increase in tumor volume occurred already four days after administration of the drug. The cancer then rapidly grew day by day, and a tendency for the cancer to spread throughout the foot was seen on the 20th day. The cancer spread to the base of the foot in some mice. Some mice were observed walking such as to drag their foot. Therefore, there was no hope for carcinostatic action nor was recovery possible, without at least continued administration of MMC or increased drug concentration.

Conversely, the iron porphyrin complex-embedded liposome exhibited strong carcinostatic action, and showed advance in the direction towards curing the cancer. Therefore, the iron porphyrin complex-embedded liposome was highly effective.

The reasons effective carcinostatic action was exhibited are considered to be as follows.

a) Because the particle size of the liposome is about 30nm, the liposome passes through the new blood vessels specifically generated in cancerous tissue by enhanced permeation and retention (EPR) effect. As a result, normal tissue is not affected, and the liposomes selectively accumulate in the cancerous tissue.

b) Because an aqueous layer attributed to the PEG chain is formed, recognition as a foreign matter by macrophages, renal glomerular filtration, and the like can be avoided. The amount of time the iron porphyrin complex remains in the blood can be extended compared to when the iron porphyrin complex is administered by itself. As a result, the concentration of effective drug in the blood can be sustained for a long period of time, and aggression against cancerous tissue increases.

c) Regarding the iron porphyrin complex-embedded/pH-sensitive liposome, after the iron porphyrin complex-embedded/pH-sensitive liposome is taken into the cell by endocytosis, the drug is sustain-released at an early stage. Therefore, very effective carcinostatic action can be seen against end-stage cancer.

d) Because the porphyrin is embedded in the surface of the liposome, rather can being incorporated therein, effective carcinostatic action can be seen Regarding iron porphyrin complex-embedded/DPPC-PEG liposome as well.

Therefore, it is clear that the iron porphyrin complex-embedded liposome has very effective carcinostatic action against B16 melanoma, with reduced side effects.

Example 2 (Early-stage cancer)

1) Animals

Five female, six-week-old IRC mice were assigned per group.

2) Sample drugs

As the sample drug (concentration) of the present invention,
iron porphyrin complex/$PLLA_{60wt\%}$-F88 micelles (5mM/0.7wt%) were used.
As the sample drug (concentration) of the comparative example,
CDDP (0.9mM) was used.

3) Cancer cells

B16 melanoma cells were used.

4) Testing method

B16 melanoma dispersed in PBS was injected into the foot pads (sole) of the mice (ICR, female, six-weeks-old), and cancer was transplanted. The amount of cancer cells injected was $1\times10^5$/mouse/0.05ml, which is 1/10 that for end-stage cancer. The site of injection was within the limited area of the foot pad, in an environment where numerous fine blood vessels, such as capillaries, are present.

Anchoring of the cancer was confirmed on the 10th day after cancer cell transplantation. The mice were separated into five mice each for the required number of groups (three groups: two groups for the sample drugs and one control groups). At the same time, the short diameter and the long diameter of the cancer cell volume in each mouse were measured every two days using calipers. The following equation was calculated:

$$[\text{tumor volume}]=1/2\times[\text{long diameter}]\times[\text{short diameter}]^2$$

In addition, administration of the sample drugs (administered amount: 0.2ml/mouse/dose) was started on the 13th day after cancer cell transplantation. Drug administration was conducted a total of four times, via tail vein every four days. The weight of each mouse was measured every two days.

5) Results

The rate of increase in tumor volume was as shown in Fig. 6 and Fig. 7.

It is clear that the iron porphyrin complex can reduce side effects and exhibit overwhelming carcinostatic action as a result of being embedded in the micelles.

Based on the above-described results, because complete remission was seen in two out of five mice, the iron porphyrin complex/$PLLA_{60wt\%}$-F88 micelles not only have overwhelming carcinostatic action, but also clearly have high anticancer action.

Although CDDP, which is a typical carcinostatic agent, is capable of suppressing increase in tumor volume by about ten times, it is not yet used for treatment. Therefore, the iron porphyrin complex/$PLLA_{60wt\%}$-F88 micelle of the present invention can be considered to have very high target orientation, or in other words targeting effect towards cancerous tissue.

In addition, when the effects of the side effects of drug administration were studied by observing the coat of each mouse in addition to weight fluctuations, shedding occurred at the slightest touch, and fur loss could be seen on the back. However, such symptoms could not be seen at all in mice that had been administered the iron porphyrin complex/$PLLA_{60wt\%}$-F88 micelles. Therefore, the low toxicity of the porphyrin drug and the accumulation of the iron porphyrin complex/$PLLA_{60wt\%}$-F88 micelles in cancerous tissue became clear.

From the above-described results, it is clear that the iron porphyrin complex/$PLLA_{60wt\%}$-F88 micelles of the present invention are highly effective against early-stage cancer.

Therefore, the polymer capsule of the present invention clearly has 10 times or more the carcinostatic action of CDDP, and MMC that are typical drugs.

In addition, as a result of the animal experiments, it is clear that the iron porphyrin complex nanocapsule of the present invention acts as a carcinostatic agent exhibiting selective effects on only cancer cells, as a replacement for carcinostatic agents such as CDDP and MMC that have significant issues regarding side effects despite its current use in clinical applications. The iron porphyrin complex nanocapsule of the present invention is also found to act as an antioxidative agent for treating diseases other than cancer, such as inflammatory conditions, neurological diseases, arteriosclerosis, and diabetes, considered to be related to reactive oxygen species.

Example 3 (Nephropathy)

1) Animals

Female, six-weeks-old HIGA/Nsc Slc mice were used as the animals. Regarding the control mince, BALB/C mice were used in adherence to experiment instructions by Japan SLC although the mice originate from ddY mice because its inbred strain is HIGA mice.

2) Sample drugs

As the sample drugs (concentration) of the present invention,
manganese porphyrin complex/pH-sensitive liposomes (5mM/36mM),
manganese porphyrin complex/DPPC-PEG liposomes (5mM/36mM), and
manganese porphyrin complex/$PLLA_{80wt\%}$-F88 vesicles (100μM/0.7wt%)
were used.

3) Testing method

0.2ml of the drug were administered to the mice (HIGA/Nsc Slc, female, six-weeks-old) every other week, via tail vein. Urinary protein and occult blood were tested by urine test once a week, and observation was conducted for a month. Pretest 10II (manufactured by Wako Pure Chemical Industries, Ltd.) was used for the urine test.

4) Results

The test results for urinary protein are shown in Table 2. The test results for occult blood are shown in Table 3.
[Table 2]

TABLE2 TEST RESULTS FOR URINARY PROTEIN IN DRUG-ADMINISTERED MICE

| NITIOXIDATIVE AGENT | URINARY PROTEIN OBSERVATION (WEEK) | | | | |
|---|---|---|---|---|---|
| | 0 | 1 | 2 | 3 | 4 |
| MANGANESE PORPHYRIN COMPLEX/DPPC-PEG LIPOSOME | +100 | +30 | +30 | +30 | +30 |
| MANHANESE PORPHYRIN COMPLEX/ph-SENSITIVE LIPOSOME | +100 | +30 | +30 | TRACE | TRACE |
| MANGANESE PORPHYRIN COMPLEX/$PLLA_{80wt\%}$-F88 VESICLE | +30 | +30 | +30 | TRACE | TRACE |
| * CONTROL : BALB(1) : +30, BALB(2) : TRACE, BALB(3) : TRACE | | | | | |

[Table 3]

TABLE3 TEST RESULTS FOR URINARY OCCULT BLOOD OF DRUG-ADMINISTERED MICE

| NITIOXIDATIVE AGENT | URINARY OCCULT BLOOD OBSERVATION (WEEK) | | | | |
|---|---|---|---|---|---|
| | 0 | 1 | 2 | 3 | 4 |
| MANGANESE PORPHYRIN COMPLEX/DPPC-PEG LIPOSOME | +++ | - | - | - | - |
| MANHANESE PORPHYRIN COMPLEX/ph-SENSITIVE LIPOSOME | - | - | - | - | |
| MANGANESE PORPHYRIN COMPLEX/$PLLA_{80wt\%}$-F88 VESICLE | ++ | - | - | - | - |
| * CONTROL : BALB(1) : -, BALB(2) : -, BALB(3) : - | | | | | |

The progress status of symptoms of IgA nephropathy differs due to individual differences depending on the mouse. Therefore, as shown in Table 2 and Table 3, how close the progress status becomes from the initial status (zero weeks) to the urine test results of the BALB mice which are the control group is used as judgment criteria.

From the above, results indicating an improvement in symptoms can be seen in all systems that had been administered the drugs. Regarding occult blood in particular, symptoms were completely cured one week after administration. Furthermore, in mice that had been administered manganese porphyrin complex/DPPC-PEG liposomes, the symptoms were improved by the following week despite particularly serious occult bleeding.

In addition, while the coat of each mouse was observed during the experiment, minimal change attributed to drug administration could be seen. Decrease in activity was also not seen. Therefore, it can be said that no particular side effects caused by porphyrin, $PLLA_{80wt\%}$-F88 vesicles, and liposome occur.

IgA nephropathy is considered to be a case where improvement can barely be seen once it occurs. However, in the present experiment, significant improvements in symptoms could be confirmed. Therefore, it is clear that the manganese porphyrin complex nanocapsule may serve as a very effective treatment drug for IgA nephropathy, and can become a new treatment drug in diseases (such as diabetes) that case large amounts of active oxygen to be produced.

## Claims

**1.** A disease treatment drug containing an iron or manganese porphyrin complex nanocapsule housing an iron or manganese porphyrin complex within a nanosized capsule, wherein:

disease of abnormal tissue having a high concentration of active oxygen can be treated by the iron or manganese porphyrin complex delivered into the abnormal tissue as a result of the nanocapsule, without affecting normal tissue having a low concentration of active oxygen, and side effects can be suppressed.

**2.** The disease treatment drug according to claim 1, wherein:

the iron porphyrin complex causes a reaction between iron that is in the center and the active oxygen within the abnormal tissue, thereby generating hydrogen peroxide, causes a reaction between the generated hydrogen peroxide and iron, thereby generating hydroxyl radicals, and killing abnormal cells by cytotoxicity of the hydroxyl radicals.

**3.** The disease treatment drug according to claim 1 or 2, wherein:

the nanocapsule is composed of a liposome or a polymer capsule.

**4.** The disease treatment drug according to any one of claims 1 to 3, wherein:

the nanocapsule has a size of 10nm to 200nm.

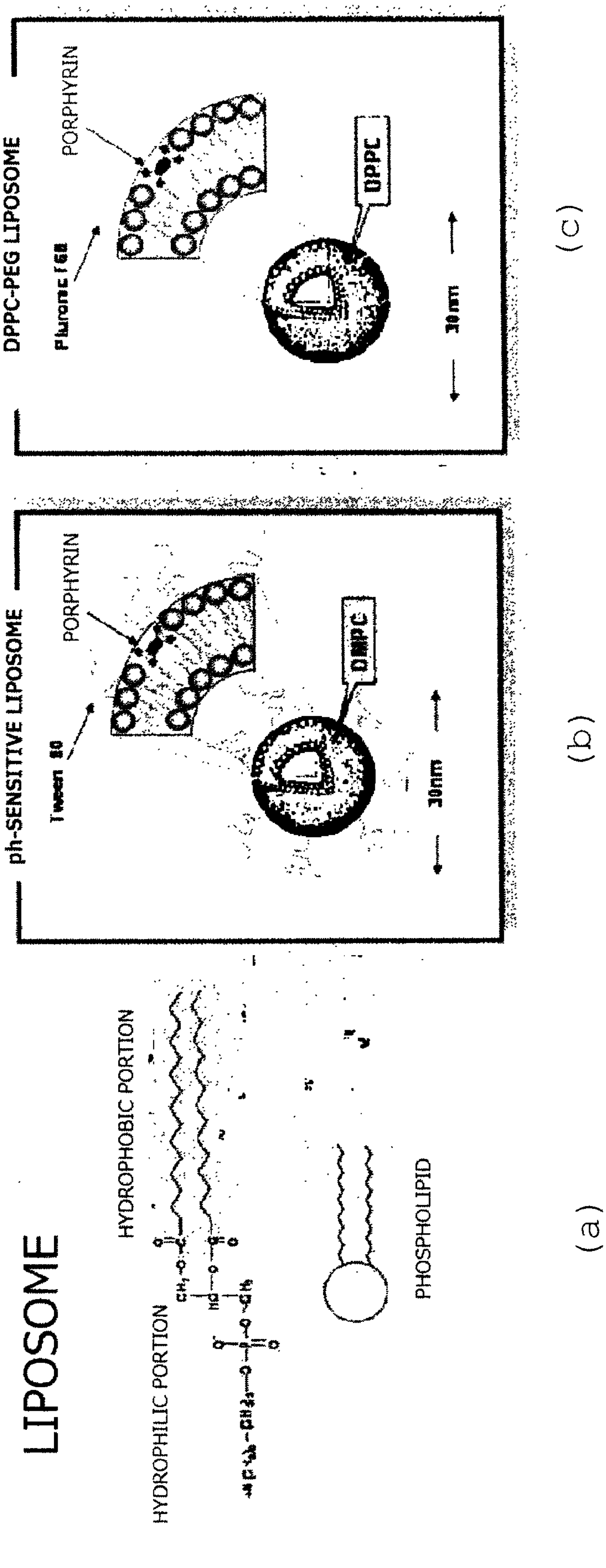
LIPOSOME
HYDROPHILIC PORTION
HYDROPHOBIC PORTION
PHOSPHOLIPID
(a)
ph-SENSITIVE LIPOSOME
Tween 80
PORPHYRIN
DMPC
30nm
(b)
DPPC-PEG LIPOSOME
Pluronic F68
PORPHYRIN
DPPC
30nm
(c)

FIG.1

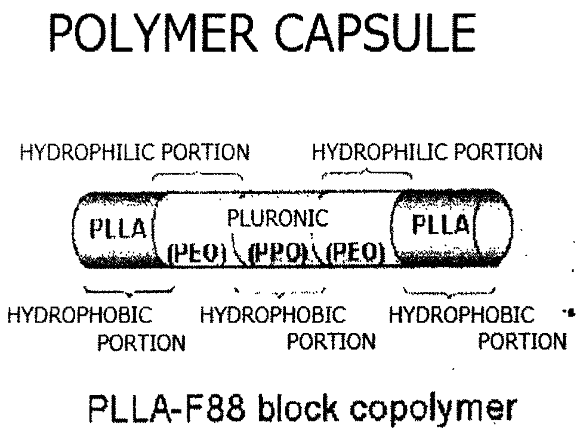
POLYMER CAPSULE
HYDROPHILIC PORTION
HYDROPHILIC PORTION
PLLA
PLURONIC
(PEO)
(PPO)
(PEO)
PLLA
HYDROPHOBIC PORTION
HYDROPHOBIC PORTION
HYDROPHOBIC PORTION
PLLA-F88 block copolymer

(a)

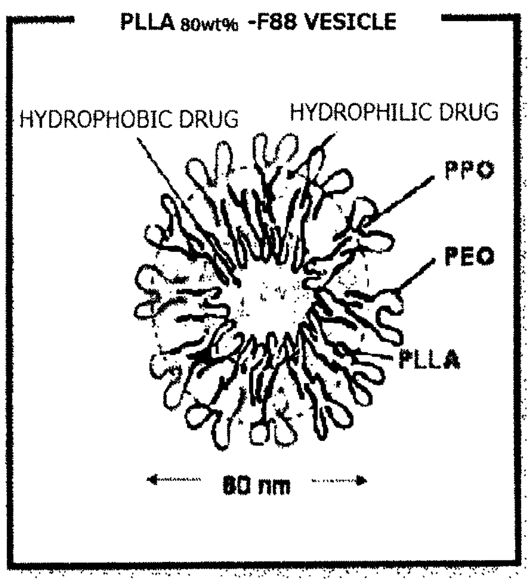
PLLA 80wt% -F88 VESICLE
HYDROPHOBIC DRUG
HYDROPHILIC DRUG
PPO
PEO
PLLA
80 nm

(b)

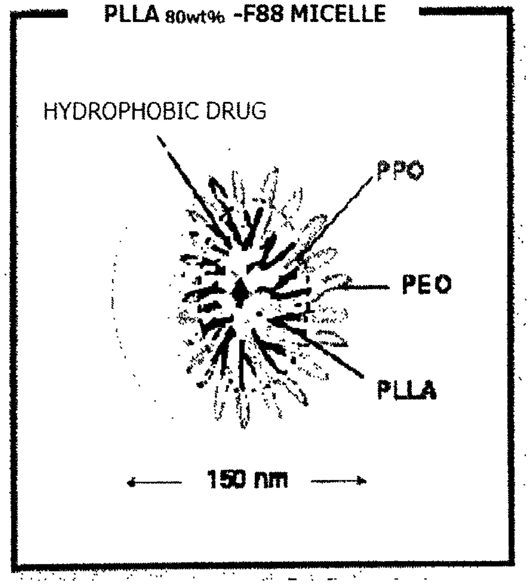
PLLA 80wt% -F88 MICELLE
HYDROPHOBIC DRUG
PPO
PEO
PLLA
150 nm

(c)

FIG.2

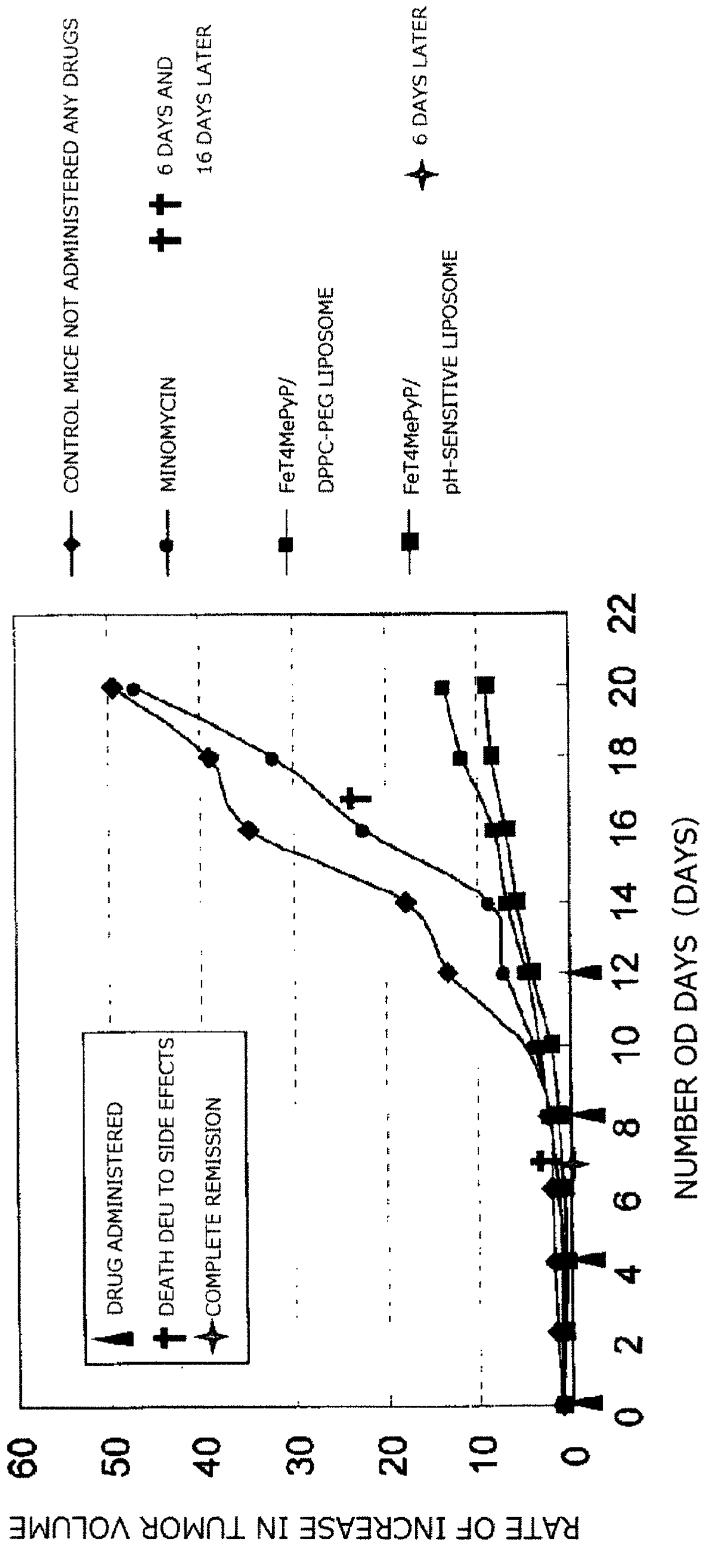
CONTROL MICE NOT ADMINISTERED ANY DRUGS
MINOMYCIN
6 DAYS AND
16 DAYS LATER
FeT4MePyP/
DPPC-PEG LIPOSOME
FeT4MePyP/
pH-SENSITIVE LIPOSOME
6 DAYS LATER
DRUG ADMINISTERED
DEATH DEU TO SIDE EFECTS
COMPLETE REMISSION
RATE OF INCREASE IN TUMOR VOLUME
0
10
20
30
40
50
60
NUMBER OD DAYS (DAYS)
0
2
4
6
8
10
12
14
16
18
20
22

FIG.3

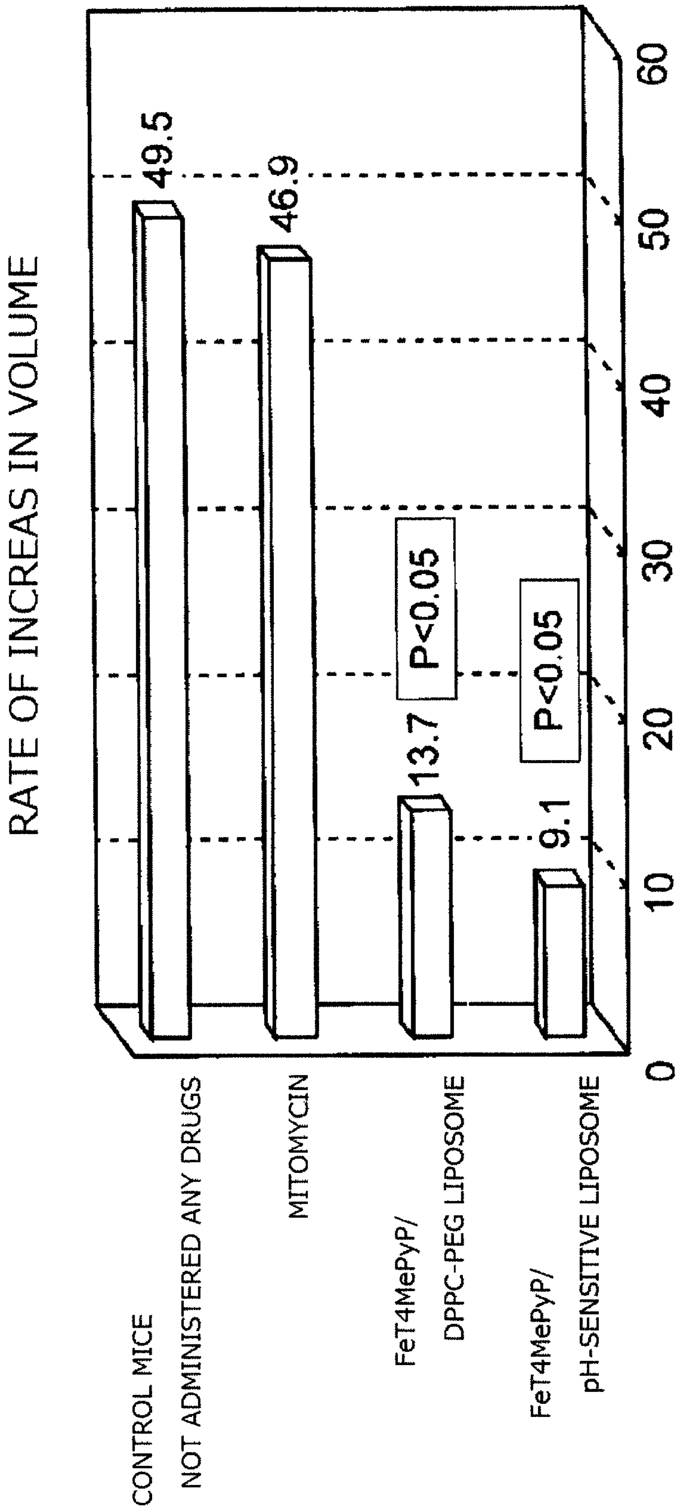
RATE OF INCREAS IN VOLUME
49.5
46.9
13.7
9.1
P<0.05
P<0.05
CONTROL MICE
NOT ADMINISTERED ANY DRUGS
MITOMYCIN
FeT4MePyP/
DPPC-PEG LIPOSOME
FeT4MePyP/
pH-SENSITIVE LIPOSOME
0
10
20
30
40
50
60

FIG. 4

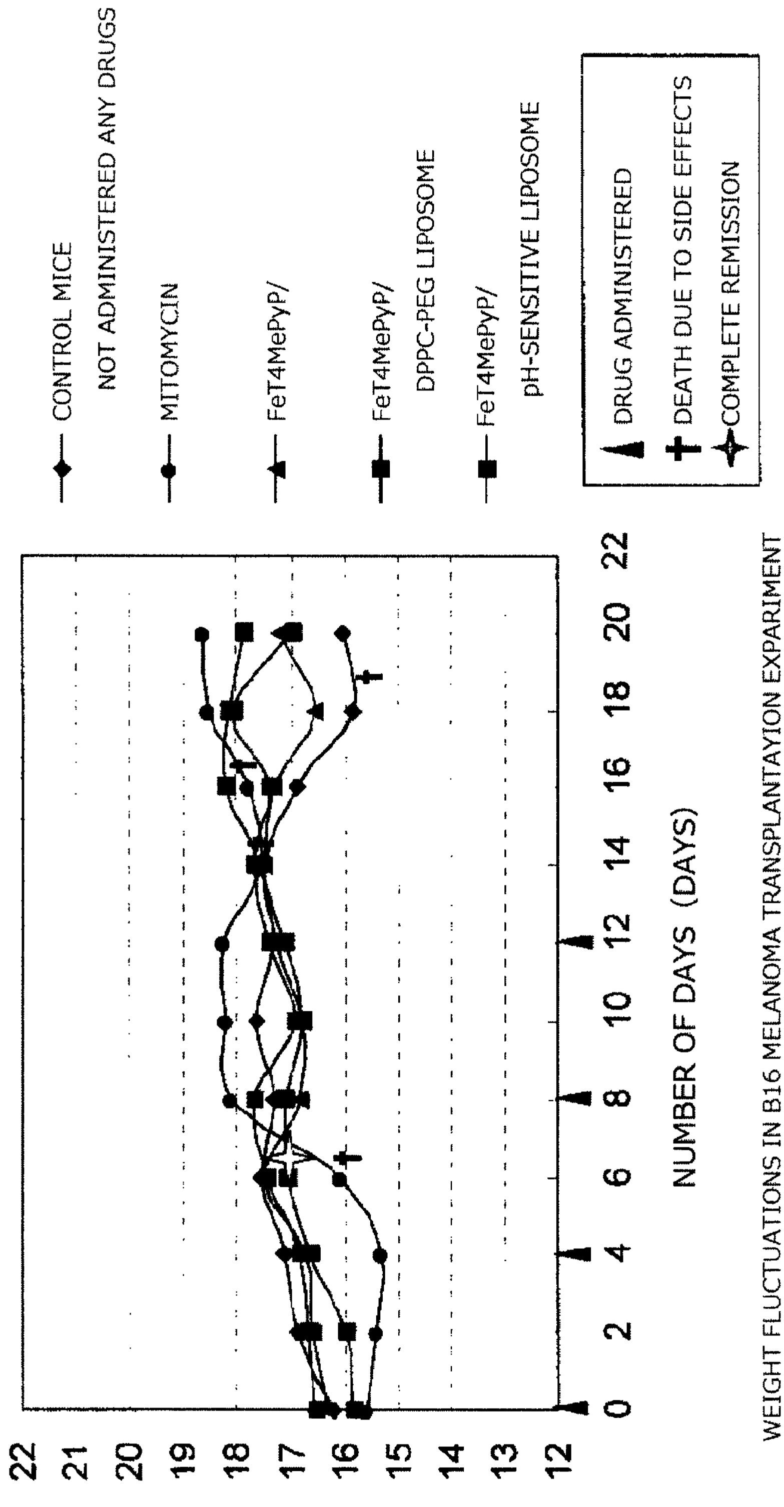


WEIGHT FLUCTUATIONS IN B16 MELANOMA TRANSPLANTAYION EXPARIMENT

FIG.5

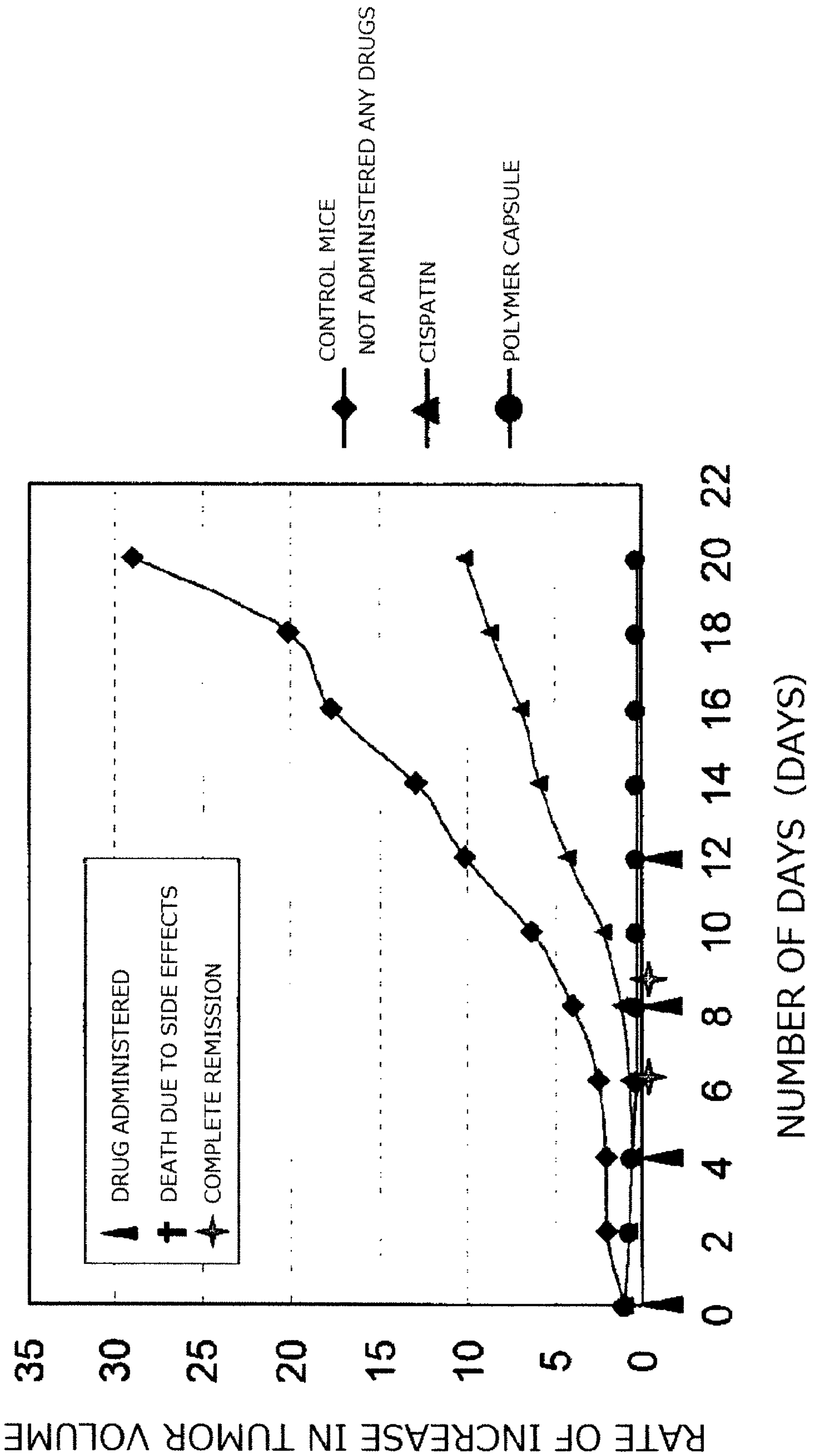
CONTROL MICE
NOT ADMINISTERED ANY DRUGS
CISPATIN
POLYMER CAPSULE
DRUG ADMINISTERED
DEATH DUE TO SIDE EFFECTS
COMPLETE REMISSION
RATE OF INCREASE IN TUMOR VOLUME
0
5
10
15
20
25
30
35
NUMBER OF DAYS (DAYS)
0
2
4
6
8
10
12
14
16
18
20
22

FIG.6

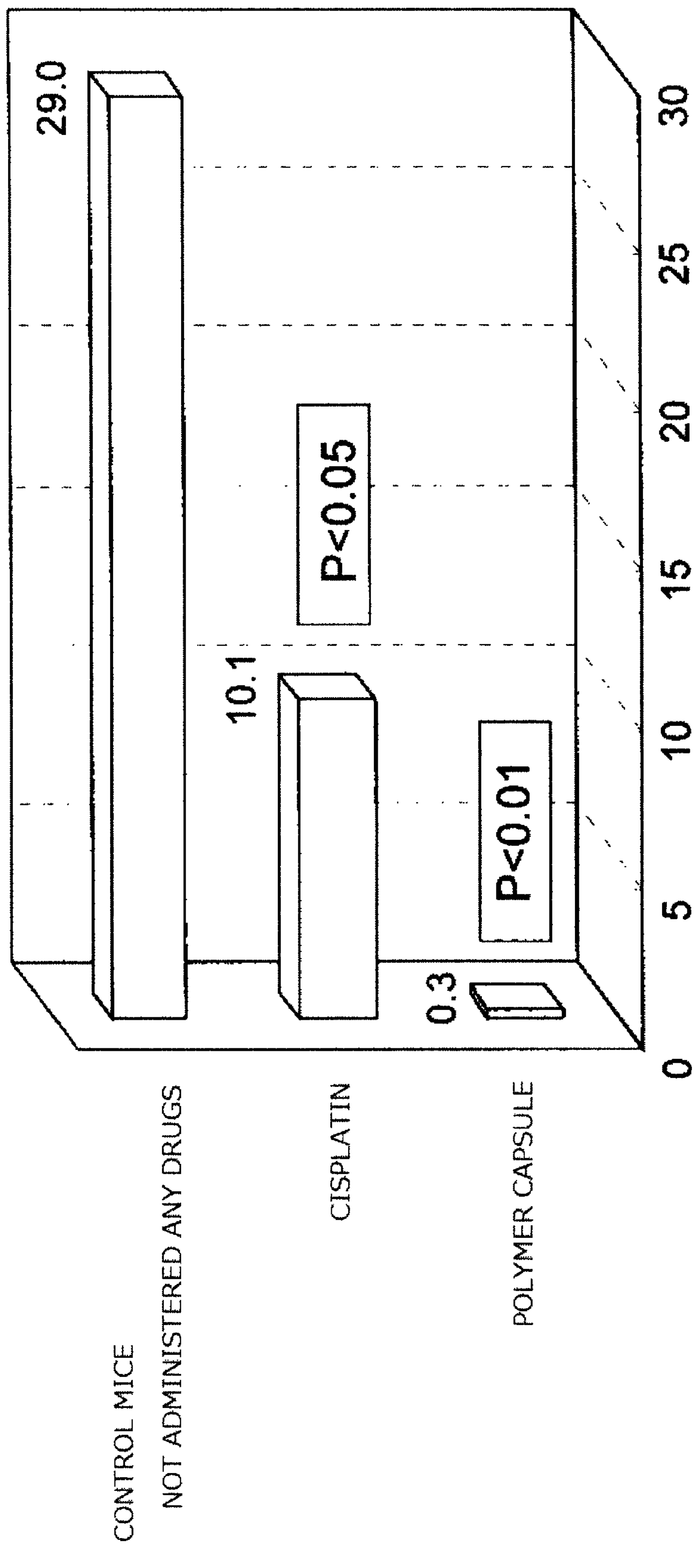
CONTROL MICE
NOT ADMINISTERED ANY DRUGS
CISPLATIN
POLYMER CAPSULE
29.0
10.1
0.3
P<0.05
P<0.01
0
5
10
15
20
25
30

FIG. 7

## INTERNATIONAL SEARCH REPORT

International application No.
PCT/JP2010/063396

**A. CLASSIFICATION OF SUBJECT MATTER**
*A61K31/555*(2006.01)i, *A61K9/127*(2006.01)i, *A61K9/51*(2006.01)i, *A61P3/10*(2006.01)i, *A61P9/10*(2006.01)i, *A61P25/00*(2006.01)i, *A61P29/00*(2006.01)i, *A61P35/00*(2006.01)i, *A61P39/06*(2006.01)i

According to International Patent Classification (IPC) or to both national classification and IPC

**B. FIELDS SEARCHED**

Minimum documentation searched (classification system followed by classification symbols)
A61K31/555, A61K9/127, A61K9/51, A61P3/10, A61P9/10, A61P25/00, A61P29/00, A61P35/00, A61P39/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| X | WO 05/084665 A1 (Makoto YUASA), 15 September 2005 (15.09.2005), claims 1 to 19; examples 1 to 11 & US 2008/0269184 A1 & EP 1731150 A1 & CN 1942184 A | 1-4 |
| X | JP 2005-41869 A (Makoto YUASA), 17 February 2005 (17.02.2005), claims 1 to 19; examples 1 to 21 & US 2005/0008687 A1 | 1-4 |
| X | Miho TAMAI et al., The Chemical Society of Japan Koen Yokoshu, 11 March 2005 (11.03.2005), vol.85th, no.2, page 1036 | 1-4 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 August, 2010 (20.08.10) | 31 August, 2010 (31.08.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005041869 A


### Non-patent literature cited in the description


- **K. KALYANASUNDARAM.** *Inorg. Chem.,* 1984, vol. 23, 2453
- **A.D. ADLER et al.** *J. Inorg. Nucl. Chem.,* 1970, vol. 32, 2443
- **T. YONETANI et al.** *J. Biol. Chem.,* 1970, vol. 245, 2988
- **P. HAMBRIGHT et al.** *Inorg. Chem.,* 1976, vol. 15, 2314
- **M. ANTIONIETTI.** *Langmuir,* 2000, vol. 16, 3214
- **D. ADLER et al.** *J. Org. Chem.,* 1967, vol. 32, 476
- **D. ADLER et al.** *Inorg. Synth.,* 1976, vol. 16, 213
- **HARRIMAN et al.** *J. Chem. Soc., Faraday. Trans.,* 1979, vol. II, 1532
- **E. TSUCHIDA ; H. NISHIDE ; H. YOKOYAMA ; R. YOUNG ; C.K. CHANG.** *Chem. Lett.,* 1984, 991